(19) Europäisches Patentamt
European Patent Office
Office européen des brevets

(11) **EP 2 197 868 B1**

(12) **EUROPEAN PATENT SPECIFICATION**

(45) Date of publication and mention
of the grant of the patent:
**27.04.2011 Bulletin 2011/17**

(51) Int Cl.:
***C07D 307/46*** *(2006.01)*     ***C10L 1/00*** *(2006.01)*

(21) Application number: **08785853.6**

(22) Date of filing: **05.09.2008**

(86) International application number:
**PCT/EP2008/007414**

(87) International publication number:
**WO 2009/030508 (12.03.2009 Gazette 2009/11)**

(54) **HYDROXYMETHYLFURFURAL ETHERS FROM SUGARS OR HMF AND MIXED ALCOHOLS**

HYDROXYMETHYLFURFURALETHER AUS ZUCKERN ODER HMF UND GEMISCHTEN ALKOHOLEN

ÉTHERS D'HYDROXYMÉTHYLFURFURAL OBTENUS À PARTIR DE SUCRES OU DE HMF ET D'ALCOOLS MIXTES

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **07.09.2007 EP 07075776**

(43) Date of publication of application:
**23.06.2010 Bulletin 2010/25**

(73) Proprietor: **Furanix Technologies B.V
1014 BV Amsterdam (NL)**

(72) Inventors:
• **GRUTER, Gerardus, Johannes, Maria
NL-2106 BA Heemstede (NL)**
• **MANZER, Leo, Ernest
Wilmington, DE 19803 (US)**

(74) Representative: **Kortekaas, Marcel C.J.A.
Exter Polak & Charlouis B.V. (EP&C)
P.O. Box 3241
2280 GE Rijswijk (NL)**

(56) References cited:
**EP-A- 1 834 950         WO-A-99/67409
WO-A-2006/063220     WO-A-2007/104514
DE-A1- 3 621 517**

• **GARVES K: "Acid catalyzed degradation of cellulose in alcohols" JOURNAL OF WOOD CHEMISTRY AND TECHNOLOGY, MARCEL DEKKER, NEW YORK, NY, US, vol. 8, no. 1, 1988, pages 121-134, XP009067962 ISSN: 0277-3813**

## Description

Technical Field

The present invention concerns a method for the manufacture of a mixture of 5-hydroxymethylfurfural (5-(hydroxymethyl)-2-furaldehyde, or HMF) ethers from biomass.

Background Art

Fuel, fuel additives and various chemicals used in the petrochemical industry are derived from oil, gas and coal, all finite sources. Biomass, on the other hand, is considered a renewable source. Biomass is biological material (including biodegradable wastes) which can be used for the production of fuels or for industrial production of e.g. fibres, chemicals or heat. It excludes organic material which has been transformed by geological processes into substances such as coal or petroleum.

Production of biomass derived products for non-food applications is a growing industry. Bio-based fuels are an example of an application with strong growing interest..

Biomass contains sugars (hexoses and pentoses) that may be converted into value added products. Current biofuel activities from sugars are mainly directed towards the fermentation of sucrose or glucose into ethanol or via complete breakdown via Syngas to synthetic liquid fuels. EP 0641 854 describes the use of fuel compositions comprising of hydrocarbons and/or vegetable oil derivatives containing at least one glycerol ether to reduce particulate matter emissions.

More recently, the acid catalysed reaction of fructose has been re-visited, creating HMF as an intermediate of great interest. Most processes investigated have the disadvantage that HMF is not very stable at the reaction conditions required for its formation. Fast removal from the water-phase containing the sugar starting material and the acid catalyst has been viewed as a solution for this problem. Researchers at the University of Wisconsin-Madison have developed a process to make HMF from fructose. HMF can be converted into monomers for plastics, petroleum or fuel extenders, or even into fuel itself. The process by prof. James Dumesic and co-workers first dehydrates the fructose in an aqueous phase with the use of an acid catalyst (hydrochloric acid or an acidic ion-exchange resin). Salt is added to salt-out the HMF into the extracting phase. The extracting phase uses an inert organic solvent that favors extraction of HMF from the aqueous phase. The two-phase process operates at high fructose concentrations (10 to 50 wt %), achieves high yields (80% HMF selectivity at 90% fructose conversion), and delivers HMF in a separation-friendly solvent (DUMESIC, James A, et al. "Phase modifiers promote efficient production of Hydroxymethylfurfural from fructose". Science. 30 juni 2006, vol.312, no.5782, p.1933-1937). Although the HMF yields from this process are interesting, the multi-solvent process has cost-disadvantages due to the relatively complex plant design and because of the less than ideal yields when cheaper and less reactive hexoses than fructose, such as glucose or sucrose, are used as a starting material. HMF is a solid at room temperature which has to be converted in subsequent steps to useful products. Dumesic has reported an integrated hydrogenolysis process step to convert HMF into dimethylfuran (DMF), which is assumed to be an interesting gasoline additive.

In WO 2006/063220 a method is provided for converting fructose into 5- ethoxymethylfurfural (EMF) at 60 °C, using an acid catalyst either in batch during 24 hours or continuously via column elution during 17 hours. Applications of EMF were not discussed.

Also in copending patent application PCT/EP2007/002145 the manufacture of HMF ethers are described, including the use of such ethers as fuel or fuel additive. Indeed, both the methyl ether and the ethyl ether (methoxymethylfurfural, or MMF; ethoxyethylfurfural or EMF) were prepared and tested. The invention of the copending patent application, however, was limited to the use of primary aliphatic alcohols, and preferably primary C1-C5 alcohols. No examples were provided with mixed alcohols e.g., a mixture of one alcohol containing at least 5 vol.% of another alcohol. Although MMF and EMF are useful as fuel or fuel additive, the inventors found that the ethers leave room for improvement, in particular when used in higher concentration blends with fuels such as gasoline, kerosene, diesel, biodiesel or green diesel. The inventors have therefore set out to overcome this shortfall.

Surprisingly, the inventors have found that ethers of HMF obtained from mixtures of alcohols have superior blending properties compared to ethers obtained from single alcohol ether analogs.

The ethers of HMF with these alcohols may be produced in a reasonable yield from hexose containing feedstock or from HMF, with reduced levels of by-product formation and in a manner that does not require cumbersome process measures (such as 2-phase systems) or lengthy process times.

Disclosure of Invention

Accordingly, the current invention provides a method for the manufacture of a mixture of 5-hydroxymethylfurfural ethers by reacting a hexose-containing starting material or HMF with mixed alcohols in the presence of an acid catalyst.

When the reaction product of the above method is used as such or when it is used as an intermediate for a subsequent conversion, the selectivity of the reaction is preferably high as the product is preferably pure. However, when the reaction product of the above method is used as a fuel, a fuel additive or as a fuel or a fuel additive intermediate, the reaction product does not necessarily need to be pure. Indeed, in the preparation of fuel and fuel additives from biomass, which in itself is a mixture of various monosaccharides, disaccharides and polysaccharides, the reaction product may contain non-interfering components such as levulinic acid derivatives and/or derivatives of pentoses and the like. For ease of reference, however, the method and the reaction product are described in terms of the reaction of a hexose-containing starting material, resulting in an ether of HMF. Also within the scope of the invention is the reaction of HMF with the branched alcohol, since HMF is believed to be produced as intermediate from the hexose-containing starting material.

The current invention also provides for the use of the reaction product made according to the present invention as fuel or as fuel additive. Fuels for blending with the product of the present invention include but are not limited to gasoline and gasoline-ethanol blends, kerosene, diesel, biodiesel (refers to a non-petroleum-based diesel fuel consisting of short chain alkyl (methyl or ethyl) esters, made by transesterification of vegetable oil, which can be used (alone, or blended with conventional petrodiesel), Fischer-Tropsch liquids (for example obtained from GTL, CTL or BTL gas-to-liquids/coal-to-liquids/biomass to liquids processes), diesel-biodiesel blends and green diesel and blends of diesel and/or biodiesel with green diesel (green diesel is a hydrocarbon obtained by hydrotreating biomass derived oils, fats, greases or pyrolysis oil; see for example the UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05GO15085). The product is a premium diesel fuel containing no sulfur and having a cetane number of 90 to 100). Fuels for blending with the product of the present invention may also include one or more other furanics, wherein the expression furanics is used to include all derivatives of furan and tetrahydrofuran. The invention also provides a fuel composition comprising a fuel element as described above and the reaction product made according to the present invention.

Mode(s) for Carrying Out the Invention

Biomass resources are well known. The components of interest in biomass are the mono-, di- or polysaccharides (hereinafter referred to as hexose-containing starting material). Suitable 6-carbon monosaccharides include but are not limited to fructose, glucose, galactose, mannose and their oxidized, reduced, etherified, esterified and amidated derivatives, e.g. aldonic acid or alditol, with glucose being the most abundant, the most economic and therefore the most preferred monosaccharide, albeit less reactive than fructose. On the other hand, the current inventors have also succeeded to convert sucrose, which is also available in great abundance. Other disaccharides that may be used include maltose, cellobiose and lactose. The polysaccharides that may be used include cellulose, inulin (a polyfructan), starch (a polyglucan) and hemi-cellulose. The polysaccharides and disaccharides are converted into their monosaccharide component(s) and dehydrated during the manufacture of the 5-HMF ether.

The mixture of alcohols used in the method of the current invention is typically a mixture of two or more monoalcohols, having a primary, secondary or tertiary hydroxyl group. Suitable alcohols have 1 to 20 carbon atoms, preferably 1 to 8 carbon atoms, and may be selected from the group comprising methanol, ethanol, and any of the isomers of propanol up to octanol. Of particular importance is the presence of a second alcohol, again having 1 to 20 carbon atoms, but being different from the first alcohol. This second alcohol needs to be present in an amount of at least 5 vol.%, preferably 10 vol.%, more preferably at least 15 vol.% in order to disturb the crystallization of the reaction product. Suitable mixtures therefore include methanol and ethanol, in a volume ratio of 5:95 to 95:5, preferably of 10:90 to 90:10, more preferably of 15:85 to 85:15. This means that "contaminated" ethanol may be used, to produce a product that even outperforms EMF in usefulness. Synthetic alcohols may be used as well, e.g., alcohols made via the Guerbet reaction (e.g., 2-ethylhexanol, prepared from butanol; "Selective synthesis of 2-ethyl-1-hexanol from n-butanol through the Guerbet reaction by using bifunctional catalysts based on copper or palladium precursors and sodium butoxide", by Carlo Carlini, Journal of Molecular Catalysis A: Chemical 212 (2004) 65-70), which therefore typically comprise a higher alcohol in combination with the lower (starting) alcohol.

Obviously, also a mixture of three or more alcohols may be used, as long as the total amount of the second and further alcohol is at least 5 vol.% in the alcohol mixture.

The amount of alcohol mixture used during the manufacture of the HMF ether is preferably at least equimolar on the hexose content of the feedstock, but typically is used in much greater excess. Indeed, the alcohol mixture (such as 50/50 butanol/ethanol) may be used as solvent or co-solvent. In such a case, a sufficient amount of alcohol is present to form the HMF ether. As the alcohols have different reactivities in the etherification reaction, the ether product ratio is not necessarily equal to the mixed alcohol feed ratio.

The acid catalyst in the method of the present invention can be selected from amongst (halogenated) organic acids, inorganic acids, Lewis acids, ion exchange resins and zeolites or combinations and/or mixtures thereof. It may be a homogeneous catalyst, but heterogeneous catalysts (meaning solid) are preferred for purification reasons. The HMF ethers can be produced with a protonic, Bronsted or, alternatively, a Lewis acid or with catalysts that have more than one of these acidic functionalities.

The protonic acid may be organic or inorganic. For instance, the organic acid can be selected from amongst oxalic acid, levulinic acid, maleic acid, trifluoro acetic acid (triflic acid), methansulphonic acid or para-toluenesulphonic acid. Alternatively, the inorganic acid can be selected from amongst (poly)phosphoric acid, sulphuric acid, hydrochloric acid, hydrobromic acid, nitric acid, hydroiodic acid, optionally generated in situ.

Certain salts may be used as catalyst, wherein the salt can be any one or more of $(NH_4)_2SO_4|SO_3$, ammonium phosphate, pyridinium chloride, triethylamine phosphate, pyridinium salts, pyridinium phosphate, pyridinium hydrochloride/hydrobromide/perbromate, DMAP, aluminium salts, Th and Zr ions, zirconium phosphate, Sc and lanthanide ions such as Sm and Y as their acetate or trifluoroactate (triflate) salt, Cr-, Al-, Ti-, Ca-, In-ions, $ZrOCl_2$, $VO(SO_4)_2$, $TiO_2$, V-porphyrine, Zr-, Cr-, Ti-porphyrine.

Lewis acids selected as dehydration catalyst can be any one of $ZnCl_2$, $AlCl_3$, $BF_3$.

Ion exchange resins can be suitable dehydration catalysts. Examples include Amberlite™ and Amberlyst™, Diaion™ and Levatit™. Other solid catalyst that may be used include natural clay minerals, zeolites, supported acids such as silica impregnated with mineral acids, heat treated charcoal, metal oxides, metal sulfides, metal salts and mixed oxides and mixtures thereof. If elevated reactions temperatures are used, as defined hereafter, then the catalyst should be stable at these temperatures.

An overview of catalysts that may be used in the method of the current invention may be found in Table 1 of the review article prepared by Mr. Lewkowski: "Synthesis, chemistry and applications of 5-hydroxymethylfurfural and its derivatives" Arkivoc. 2001, p.17-54.

The amount of catalyst may vary, depending on the selection of catalyst or catalyst mixture. For instance, the catalyst can be added to the reaction mixture in an amount varying from 0.01 to 40 mole % drawn on the hexose content of the biomass resource, preferably from 0.1 to 30 mole %, more preferably from 1 to 20 mole %.

In the preferred embodiment, the catalyst is a heterogeneous catalyst.

The temperature at which the reaction is performed may vary, but in general it is preferred that the reaction is carried out at a temperature from 50 to 300 degrees Celsius, preferably from 125 to 250 degrees Celsius, more preferably from 150 to 225 degrees Celsius. In general, temperatures higher than 300 are less preferred as the selectivity of the reaction reduces and as many by-products occur, inter alia caramelisation of the sugar. Performing the reaction below the lowest temperature is also less preferable because of the low reaction rate. If the reactions are carried out above the boiling temperature of water, then the reactions are preferably carried out under pressure, e.g., 10 bar nitrogen or higher.

The hexose-containing starting material is typically dissolved or suspended in a solvent which can be the mixed alcohol reactant, in order to facilitate the reaction. The solvent system may be one or more selected from the group consisting of water, sulfoxides, preferably DMSO, ketones, preferably methyl ethylketone, methylisobutylketone and acetone, ethylene glycol ethers, preferably diethyleneglycol dimethyl ether (diglyme). Also so-called ionic liquids may be used. The latter refers to a class of inert ionic compounds with a low melting point, which may therefore be used as solvent. Examples thereof include e.g., 1-H-3-methyl imidazolium chloride, discussed in "Dehydration of fructose and sucrose into 5-hydroxymethylfurfural in the presence of 1-H-3-methyl imidazolium chloride acting both as solvent and catalyst", by Claude Moreau et al, Journal of Molecular Catalysis A: Chemical 253 (2006) 165-169.

A sufficient amount of solvent is preferably present to dissolve or suspend the starting material and to limit undesired side-reactions.

The method of the current invention may be carried out in a batch process or in a continuous process, with or without recycle of (part of) the product stream to control the reaction temperature (recycle via a heat exchanger). For instance, the method of the invention can be performed in a continuous flow process. In such method, homogenous catalysts may be used and the residence time of the reactants in the flow process is between 0.1 second and 10 hours, preferably from 1 second to 1 hours, more preferably from 5 seconds to 20 minutes.

Alternatively, the continuous flow process may be a fixed bed continuous flow process or a reactive (catalytic) distillation process with a heterogeneous acid catalyst. To initiate or regenerate the heterogeneous acid catalyst or to improve performance, an inorganic or organic acid may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100 $min^{-1}$.

The above process results in a stable HMF ether, which can then be used as such or be converted into a further derivative before being used as fuel and/or as fuel additive. The inventors are of the opinion that some of the products prepared by the method of the current invention are actually new. Thus, the mixed ethers made with mixed alcohols, are new and are excellent fuel components or fuel additives. Since these alcohols may be made from biomass, this might open a class of products that are fully biomass-derived. Accordingly, these new ethers are claimed as well.

The mixed HMF ethers of the invention can also be used as or can be converted to compounds that can be used as solvent, as monomer in a polymerization (such as 2,5-furan dicarboxylic acid or FDCA), as fine chemical or pharmaceutical intermediate, or in other applications. Oxidation of the mixed HMF ethers using an appropriate catalyst under appropriate conditions such as for example described for p-xylene with a NHPI/Co(OAC)$_2$/MnOAC)$_2$ catalyst system in Adv. Synth. Catal. 2001, 343, 220-225 or such as described for HMF with a Pt/C catalyst system at pH < 8 in EP 0 356 703 or or such as described for HMF with a Pt/C catalyst system at pH > 7 in FR 2 669 634, all with air as an oxidant, resulted in the formation of 2,5- Furan dicarboxylic acid (FDCA).

The invention further concerns the use of the mixed HMF ethers prepared by the method of the current invention as fuel and/or as fuel additive. Of particular interest is the use of the mixed ethers in diesel, biodiesel or "green diesel", given its (much) greater solubility therein than ethanol. Conventional additives and blending agents for diesel fuel may be present in the fuel compositions of this invention in addition to the above mentioned fuel components. For example, the fuels of this invention may contain conventional quantities of conventional additives such as cetane improvers, friction modifiers, detergents, antioxidants and heat stabilizers, for example. Especially preferred diesel fuel formulations of the invention comprise diesel fuel hydrocarbons and HMF ether as above described together with peroxidic or nitrate cetane improvers such as ditertiary butyl peroxide, amyl nitrate and ethyl hexyl nitrate for example.

Examples are enclosed to illustrate the method of the current invention and the suitability of the products prepared therefrom as fuel. The examples are not meant to limit the scope of the invention.

**Example 1. Mixed ether formation - batch experiment**

In a 7.5 ml batch reactor, 0.053 mmol fructose in methanol/ethanol/n-butanol/water 23/45/23/9 v/v/v/v, was reacted for 1 hour at a temperature of 150 degrees Celsius with 9 mg acid catalyst. Four furan peaks were observed in the UV spectrum. Mass spectrometry identified these products as HMF, methoxymethyl furfural (MMF), ethoxymethyl furfural (EMF) and n-butoxymethylfurfural (nBMF). Also levulinic acid (LA) was found. Selectivities and conversions for catalysts used in this ex-ample can be found in table below.

Conversion of substrate, selectivity and yield of furan derivatives were calculated according to the following formulae:

$$X = 100 * m_{r\ substrate} / m_{0\ substrate}$$

| | |
|---|---|
| X | conversion (%) |
| $m_{r\ substrate}$ | amount of reacted substrate (mg) |
| $m_{0\ substrate}$ | amount of substrate in feed (mg) |

$$S_{compound} = 100 * n_{r\ substrate} / n_{0\ substrate}$$

| | |
|---|---|
| $S_{compound}$ | selectivity to compound (%) |
| $n_{r\ substrate}$ | moles of substrate reacted |
| $n_{0\ substrate}$ | moles of substrate in feed |

$$Yield = 100 * n_{product} / n_{0\ substrate}$$

| | |
|---|---|
| Yield | yield (%) |
| $n_{produd}$ | moles of product formed |

Table 1. Conversion and selectivities for the dehydration of fructose in the presence of mixed alcohols in batch.

| Cat | Conversion [%] | s HMF [%] | s EMF [%] | s MMF [%] | s nBuMF [%] | s LA[%] |
|---|---|---|---|---|---|---|
| CrCl2 | 91.6 | 9.7 | 10.5 | 10.0 | 2.8 | 4.8 |
| Ce(IV) triflate hydrate | 97.5 | 2.4 | 13.5 | 10.2 | 4.4 | 2.7 |
| Amberlyst36Dry | 99.7 | 4.2 | 17.6 | 14.5 | 4.2 | 6.6 |
| Amberlyst36Wet | 99.8 | 10.2 | 18.5 | 16.5 | 4.1 | 14.9 |

**Example 2. Mixed ethers formation from fructose (or glucose) and mixed alcohols-continuous flow experiment**

A 1.25 wt% solution of sugar (Frc or Glc) in methanol/ethanol/n-butanol/water 23/45/23/9 v/v/v/v, was flowed through a fixed bed (200 μl) of a catalyst at 190 °C. Flow rates were selected such to achieve a space velocity of 0.25 or 0.5 min$^{-1}$, i.e. a contact time of 2 or 4 min.

In all cases tBMF was detected by HPLC and identified by LC-MS (CI) in the effluent stream. Conversion of substrate, selectivity and yield of furan derivatives were calculated using the same method as for the batch reactions.

Table 2. Conversion and selectivities for the dehydration of fructose in the presence of mixed alcohols in flow.

| Substrate | Cat | Contact time [min] | Space velocity [min-1] | TOS [min] | Conversion [%] | sEthers [%] | S EMF [%] | S MMF [%] | S nBuMF [%] | S HMF [% |
|---|---|---|---|---|---|---|---|---|---|---|
| Frc | Bentonite | 0.7 | 1.5 | 80 | 84.6 | 33.7 | 13.4 | 18.1 | 2.2 | 54.4 |
| Frc | Bentonite | 4.0 | 0.3 | 80 | 98.8 | 26.6 | 1.4 | 19.9 | 5.3 | 4.4 |
| Frc | $H_2SO_4$ | 11.3 | 0.1 | 105 | 87.6 | 40.3 | 15.1 | 21.5 | 3.7 | 3.0 |
| Frc | $H_2SO_4$ | 13.2 | 0.1 | 105 | 78.9 | 79.1 | 29.3 | 42.7 | 7.2 | 7.3 |
| Glc | Bentonite | 3.1 | 0.3 | 70 | 97.7 | 30.6 | 11.1 | 16.5 | 2.9 | 3.3 |
| Glc | Bentonite | 0.4 | 2.5 | 70 | 87.7 | 21.4 | 8.4 | 11.5 | 1.5 | 18.1 |
| Glc | Bentonite | 3.1 | 0.3 | 80 | 97.6 | 33.5 | 12.2 | 18.1 | 3.2 | 3.6 |
| Glc | Bentonite | 0.4 | 2.5 | 80 | 87.7 | 20.4 | 8.0 | 11.0 | 1.4 | 18.6 |

**Example 3**

In a batch experiment, 0.36 mmol of substrate (glucose, fructose or HMF) and 6.5 mg of a solid acid catalyst were mixed in a reactor coated inside with Teflon. 0.8 ml of alcohols mixture (Methanol, Ethanol and n-Butanol with 1/2/1 volume ratio) was added and pressurized at 12.5 bar with nitrogen. The reaction was performed at different temperatures and different reaction time. Four main peaks were observed in the UV spectrum and identified as HMF, 5-(ethoxymethyl)furfural (EMF), 5-(methoxymethyl)furfural (MMF) and 5-(butoxymethyl)furfural nBuMF. In this experiment and in the experiment of Example 4, the selectivity was calculated slightly different based on the formula:

$$\text{Selectivity} = 100 * n_t(\text{product}) / [n_0(\text{substrate}) - n_t(\text{substrate})]$$

Where:

$n_0$- the initial number of moles
$n_t$- the number the moles of a compound at time "t".

The results are listed in Table 3.

Table 3. Conversion (Conv.) of glucose or fructose and selectivities (S) to furan compounds, in the presence of different solid acid catalysts at 1 h and 150 °C.

| Subst. | Catalyst | Conv. (%) | s HMF (%) | s EMF (%) | s MMF (%) | s nBuMF (%) |
|---|---|---|---|---|---|---|
| Fructose | Zeolite β | 81.8 | 2.9 | 14.9 | 14.9 | 5.2 |
| | Zeolite HY 15 | 74.2 | 7 | 14.2 | 14.2 | 4.4 |
| | HY 5 | 81.8 | 16 | 10.5 | 12.6 | 2.1 |
| | Montmorillonite K 5 | 84.4 | 9.5 | 14.5 | 15.5 | 3.9 |
| | CrCl$_2$ | 99.4 | 12.6 | 15.7 | 14.8 | 5 |
| | Amberlyst 36 dry | 98.2 | 11.4 | 17.4 | 19.1 | 4.9 |
| | Amberlyst 36 wet | 93.1 | 17.5 | 14.4 | 16.9 | 3.6 |
| | Amberlyst 70 | 85.8 | 22.2 | 12.5 | 14 | 3.3 |
| Glucose | CrCl$_2$ | 100 | 7.8 | 11.8 | 10.2 | 2.7 |
| | Zeolite HY 5 | 100 | 4.3 | 4.8 | 5.4 | 0.8 |

**Example 4**

In a batch experiment, 0.36 mmol of substrate (glucose, fructose or HMF) and 6.5 mg of a solid acid catalyst were mixed in a reactor coated inside with Teflon. 0.8 ml of alcohols mixture (Ethanol, Isobutanol and n-Hexanol, with 4/2/1 volume ratio) was added and pressurized at 12.5 bar with nitrogen (12.5 bar). The etherification was carried out at different temperatures and different reaction time. Four main peaks were observed in the UV spectrum and identified as HMF, 5-(ethoxymethyl)furfural (EMF), 5-(isobutoxymethyl)furfural (iBuMF) and 5-(hexoxymethyl)furfural (nHexMF). The results are listed in Table 4.

Table 4. Conversion (Conv.) of HMF and selectivities (S) to 5-(alkoxymethyl)furfural, in the presence of different solid acid catalysts at 3 h reaction time and 100 °C

| Catalyst | Conv. (%) | S EMF (%) | S iBuMF (%) | S nHexMF (%) |
|---|---|---|---|---|
| Amberlyst 70 | 81.2 | 48.8 | 24.4 | 2.8 |
| Dowex Dr-2030 | 94.9 | 57.9 | 21.2 | 1.9 |

(continued)

| Catalyst | Conv. (%) | S EMF (%) | S iBuMF (%) | S nHexMF (%) |
|---|---|---|---|---|
| Lewatit K-2629, H + Form | 90.7 | 61.0 | 21.8 | 2.1 |
| Montmorillonite K 5 | 88.5 | 55.9 | 31.3 | 3.3 |
| Nafion | 98.7 | 49.7 | 32.3 | 4.6 |
| Amberlyst36Wet | 82.3 | 54.4 | 22.0 | 2.3 |
| Zeolite H-USY SAR15 | 79.6 | 51.8 | 29.2 | 4.6 |

**Analytical Method**

The reaction products were quantified with the aid of HPLC-analysis with an internal standard (saccharine, Sigma Aldrich). An Agilent 1100 series chromatograph, equipped with UV and ELSD detectors, was used. Stationary phase was reverse phase C18 (Sunfire 3.5 $\mu$m, 4.6x100mm, Waters) column. A gradient elution at a constant flow 0.6 ml/min and temperature 40 °C was used according to the following scheme.

| Time | H2O(vol%) | MeOH (vol%) | MeCN (vol%) | Flow (ml/min) | T (C) |
|---|---|---|---|---|---|
| Initial | 95 | 0 | 5 | 1 | 40 |
| 1 | 89 | 3 | 8 | 1 | 40 |
| 8 | 25 | 3 | 72 | 1 | 40 |

**Example 5. diesel fuel applications**

Fuel solubility

Fuel solubility is a primary concern for diesel fuel applications. Not all highly polar oxygenates have good solubility in the current commercial diesel fuels. Results show that in the 5 vol%, in the 25 vol% and in the 40 vol% blends of total HMF ether content, obtained from a mixed alcohol etherification method according to the present invention, with commercial diesel, both liquid blend components are completely miscible. In a comparative set of experiments it was shown that ethoxymethylfurfural (EMF) is completely miscible in a 5 vol% blend with commercial diesel, but that phase separation occurs with the 25 vol% and with the 40 vol% blends of EMF and diesel.

References

- DUMESIC, James A, et al. "Phase modifiers promote efficient production of Hydroxymethylfurfural from fructose" . Science. 30 June 2006, vol.312, no.5782, p.1933-1937.
- WO 2006/063220
- Chapter 15 of Advanced Organic Chemistry, by Jerry March, and in particular under reaction 5-4. (3rd ed., © 1985 by John Wiley & Sons, pp. 684-685).
- LEWKOWSKI, Jaroslaw. Synthesis, chemistry and applications of 5-hydroxymethylfurfural and its derivatives. Arkivoc. 2001, p.17-54.
- MOREAU, Claude, et al. "Dehydration of fructose and sucrose into 5-hydroxymethylfurfural in the presence of 1-H-3-methyl imidazolium chloride acting both as solvent and catalyst", Journal of Molecular Catalysis A: Chemical 253 (2006) p. 165-169.
- EP 0641 854
- UOP report OPPORTUNITIES FOR BIORENEWABLES IN OIL REFINERIES FINAL TECHNICAL REPORT, SUBMITTED TO: U.S. DEPARTMENT OF ENERGY (DOE Award Number: DE-FG36-05G015085))
- Adv. Synth. Catal. 2001, 343, 220-225
- EP 0 356 703
- FR 2 669 634

**Claims**

1. Method for the manufacture of a mixture of 5-hydroxymethylfurfural ethers by reacting a hexose-containing starting material or HMF with a mixture of at least two dissimilar alcohols, with the second alcohol present in an amount of at least 5 vol.% on the alcohol mixture, in the presence of an acid catalyst.

2. Method according to claim 1, wherein the alcohols making up the mixture are C1-C20 alcohols.

3. Method according to claim 1, wherein the alcohols are mixtures obtained from a Fischer-Tropsch alcohol synthesis process

4. Method according to claim 1, wherein the alcohols are mixtures obtained from a Guerbet alcohol synthesis process

5. Method according to claim 1 to 4, wherein the acid catalyst is selected from the group consisting of homogeneous or heterogeneous catalysts selected from solid organic acids, inorganic acids, salts, Lewis acids, ion exchange resins, zeolites or mixtures and/or combinations thereof.

6. Method according to any one of the claims 1 to 5, wherein the reaction is performed at a temperature from 50 to 300 degrees Celsius, preferably from 125 to 250 degrees Celsius, more preferably from 150 to 225 degrees Celsius.

7. Method according to any one of the claims 1 to 6, wherein the hexose-containing starting material is used and wherein the hexose starting material is selected from the group of

   • starch, amylose, galactose, cellulose, hemi-cellulose,
   • glucose-containing disaccharides such as sucrose, maltose, cellobiose, lactose, preferably glucose-containing disaccharides, more preferably sucrose,
   • glucose or fructose.

8. Method according to any one of the claims 1 to 7, wherein the starting material is 5-(hydroxymethyl)furfural.

9. Method according to any one of the claims 1 to 7, wherein the starting material comprises glucose, fructose, galactose and mannose and their oxidized (aldonic acid) or reduced (alditol) derivatives or mixtures thereof.

10. Method according to any one of the claims 1 to 7 wherein the starting material is an esterified, etherified monosaccharide or an amido sugar.

11. Method according to any one of the claims 1 to 10, performed in the presence of a solvent, wherein the solvent or solvents are selected form the group consisting of water, sulfoxides, preferably DMSO, ketones, preferably methyl ethylketone, ionic liquids, methylisobutylketone and/or acetone esters, ethers, preferably ethylene glycol ethers, more preferably diethyleneglycol dimethyl ether (diglyme) or the reactant olefin and mixtures thereof.

12. Method according to any one of the claims 1 to 11, wherein the method is performed in a continuous flow process, and wherein preferably

   a) the residence time in the flow process is between 0.1 second and 10 hours, preferably from 1 second to 1 hours, more preferably from 5 seconds to 20 minutes;
   b) the continuous flow process is a fixed bed continuous flow process;
   c) the fixed bed comprises a heterogeneous acid catalyst;.
   d) the continuous flow process is a reactive distillation or a catalytic distillation process, and
   e) in addition to a heterogeneous acid catalyst, an inorganic or organic acid catalyst is added to the feed of the fixed bed or catalytic distillation continuous flow process.

13. Method according to claim 12, wherein the liquid hourly space velocity ("LHSV") is from 1 to 1000, preferably from 5 to 500, more preferably from 10 to 250 and most preferably from 25 to 100.

14. Use of the ether mixture produced by the method of any one of claims 1-13 as fuel or fuel additive.

15. A fuel or fuel composition comprising the ether mixture produced by the method of any one of claims 1-13 as fuel component, optionally blended with one or more of gasoline and gasoline-ethanol blends, kerosene, diesel, biodiesel (a non-petroleum-based diesel fuel consisting of short chain alkyl (methyl or ethyl) esters, made by transesterification of vegetable oil), Fischer-Tropsch liquids, diesel-biodiesel blends and green diesel (a hydrocarbon obtained by hydrotreating biomass derived oils, fats, greases or pyrolysis oil) and blends of diesel and/or biodiesel with green diesel and other derivatives of furan and tetrahydrofuran.

**Patentansprüche**

1. Verfahren zur Herstellung eines Gemisches aus 5-Hydroxymethylfurfuralethern durch Umsetzen eines Hexose enthaltenden Ausgangsmaterials oder HMF mit einem Gemisch aus wenigstens zwei verschiedenen Alkoholen, wobei der zweite Alkohol in einer Menge von wenigstens 5 Volumenprozent, bezogen auf das Alkoholgemisch, vorliegt, in Gegenwart eines sauren Katalysators.

2. Verfahren gemäß Anspruch 1, wobei die Alkohole, die das Gemisch ausmachen, C1-C20-Alkohole sind.

3. Verfahren gemäß Anspruch 1, wobei die Alkohole Gemische sind, die aus einem Fischer-Tropsch-Alkohol-Syntheseverfahren erhalten werden.

4. Verfahren gemäß Anspruch 1, wobei die Alkohole Gemische sind, die aus einem Guerbet-Alkohol-Syntheseverfahren erhalten werden.

5. Verfahren gemäß Anspruch 1 bis 4, wobei der saure Katalysator aus der Gruppe, bestehend aus homogenen oder heterogenen Katalysatoren, ausgewählt aus festen organischen Säuren, anorganischen Säuren, Salzen, Lewis-Säuren, Ionenaustauschharzen, Zeolithen oder Gemischen und/oder Kombinationen davon, ausgewählt wird.

6. Verfahren gemäß einem der Ansprüche 1 bis 5, wobei die Reaktion bei einer Temperatur von 50 bis 300 Grad Celsius, vorzugsweise von 125 bis 250 Grad Celsius, bevorzugter von 150 bis 225 Grad Celsius, durchgeführt wird.

7. Verfahren gemäß einem der Ansprüche 1 bis 6, wobei das Hexose enthaltende Ausgangsmaterial verwendet wird und wobei das Hexose-Ausgangsmaterial ausgewählt ist aus der Gruppe aus

   • Stärke, Amylose, Galactose, Cellulose, Hemicellulose,
   • Glucose enthaltenden Disacchariden, zum Beispiel Saccharose, Maltose, Cellobiose, Lactose, vorzugsweise Glucose enthaltenden Disacchariden, bevorzugter Saccharose,
   • Glucose oder Fructose.

8. Verfahren gemäß einem der Ansprüche 1 bis 7, wobei das Ausgangsmaterial 5-(Hydroxymethyl)furfural ist.

9. Verfahren gemäß einem der Ansprüche 1 bis 7, wobei das Ausgangsmaterial Glucose, Fructose, Galactose und Mannose und ihre oxidierten (Aldonsäure-) oder reduzierten (Alditol-) Derivate oder Gemische davon umfasst.

10. Verfahren gemäß einem der Ansprüche 1 bis 7, wobei das Ausgangsmaterial ein verestertes, verethertes Monosaccharid oder ein Amidozucker ist.

11. Verfahren gemäß einem der Ansprüche 1 bis 10, das in Gegenwart eines Lösungsmittels durchgeführt wird, wobei das Lösungsmittel oder die Lösungsmittel aus der Gruppe, bestehend aus Wasser, Sulfoxiden, vorzugsweise DMSO, Ketonen, vorzugsweise Methylethylketon, ionischen Flüssigkeiten, Methylisobutylketon und/oder Aceton, Estern, Ethern, vorzugsweise Ethylenglykolethern, bevorzugter Diethylenglykoldimethylether (Diglyme) oder dem Reaktanten-Olefin und Gemischen davon, ausgewählt wird/werden.

12. Verfahren gemäß einem der Ansprüche 1 bis 11, wobei das Verfahren in einem kontinuierlichen Strömungsverfahren durchgeführt wird und wobei vorzugsweise

   a) die Verweilzeit im Strömungsverfahren zwischen 0,1 Sekunden und 10 Stunden, vorzugsweise von 1 Sekunde bis 1 Stunde, bevorzugter von 5 Sekunden bis 20 Minuten, ist;
   b) das kontinuierliche Strömungsverfahren ein kontinuierliches Festbett-Strömungsverfahren ist;
   c) das Festbett einen heterogenen sauren Katalysator umfasst;

d) das kontinuierliche Strömungsverfahren eine reaktive Destillation oder ein katalytisches Destillationsverfahren ist und

e) zusätzlich zu einem heterogenen sauren Katalysator ein anorganischer oder organischer Säurekatalysator der Beschickung des kontinuierlichen Festbett- oder katalytischen Destillationsströmungsverfahren zugesetzt wird.

13. Verfahren gemäß Anspruch 12, wobei die stündliche Raumgeschwindigkeit der Flüssigkeit (liquid hourly space velocity ("LSHV")) 1 bis 1.000, vorzugsweise 5 bis 500, bevorzugter 10 bis 250 und am bevorzugtesten 25 bis 100 ist.

14. Verwendung des Ethergemisches, das durch das Verfahren gemäß einem der Ansprüche 1-3 hergestellt wird, als Kraftstoff oder Kraftstoffadditiv.

15. Kraftstoff oder Kraftstoffzusammensetzung, die das Ethergemisch, das durch das Verfahren gemäß einem der Ansprüche 1-13 hergestellt wird, als Kraftstoffkomponente umfasst, gegebenenfalls vermischt mit einem oder mehreren von Benzin und Benzin-Ethanol-Mischungen, Kerosin, Diesel, Biodiesel (ein Dieselkraftstoff, der nicht auf Erdöl basiert ist, der aus kurzkettigen Alkyl- (Methyl- oder Ethyl-) Estern, hergestellt durch Umesterung von Pflanzenöl, besteht), Fischer-Tropsch-Flüssigkeiten, Diesel-Biodiesel-Mischungen und grünem Diesel (ein Kohlenwasserstoff, der durch Hydrobehandlung von von Biomasse stammenden Ölen, Fetten, Fetten oder Pyrolyseöl, erhalten wird) und Mischungen von Diesel und/oder Biodiesel mit grünem Diesel und anderen Derivaten von Furan und Tetrahydrofuran.

**Revendications**

1. Procédé de fabrication d'un mélange de 5-hydroxyméthylfurfural éthers par réaction d'une matière de départ contenant de l'hexose ou de HMF avec un mélange d'au moins deux alcools dissemblables, le second alcool étant présent dans une quantité d'au moins 5 % en volume du mélange d'alcools, en présence d'un catalyseur acide.

2. Procédé selon la revendication 1, dans lequel les alcools constituant le mélange sont des alcools en $C_1$ à $C_{20}$.

3. Procédé selon la revendication 1, dans lequel les alcools sont des mélanges obtenus à partir d'un procédé de synthèse d'alcool de Fischer-Tropsch.

4. Procédé selon la revendication 1, dans lequel les alcools sont des mélanges obtenus à partir d'un procédé de synthèse d'alcool de Guerbet.

5. Procédé selon les revendications 1 à 4, dans lequel le catalyseur acide est choisi dans le groupe constitué par les catalyseurs homogènes ou hétérogènes choisis parmi les acides organiques solides, les acides inorganiques, les sels, les acides de Lewis, les résines échangeuses d'ions, les zéolites ou leurs mélanges et/ou combinaisons.

6. Procédé selon l'une quelconque des revendications 1 à 5, dans lequel la réaction est effectuée à une température de 50 à 300 degrés Celsius, de préférence de 125 à 250 degrés Celsius, de manière davantage préférée de 150 à 225 degrés Celsius.

7. Procédé selon l'une quelconque des revendications 1 à 6, dans lequel la matière de départ contenant de l'hexose est utilisée, et dans lequel la matière de départ contenant de l'hexose est choisie dans le groupe constitué par

• l'amidon, l'amylose, le galactose, la cellulose, l'hémi-cellulose,
• les disaccharides contenant du glucose tels que le saccharose, le maltose, le cellobiose, le lactose, de préférence les disaccharides contenant du glucose, de manière davantage préférée le saccharose,
• le glucose ou le fructose.

8. Procédé selon l'une quelconque des revendications 1 à 7, dans lequel la matière de départ est le 5-(hydroxyméthyl) furfural.

9. Procédé selon l'une quelconque des revendications 1 à 7, dans lequel la matière de départ comprend du glucose, du fructose, du galactose et du mannose et leurs dérivés oxydés (acide aldonique) ou réduits (alditol) ou leurs mélanges.

**10.** Procédé selon l'une quelconque des revendications 1 à 7, dans lequel la matière de départ est un monosaccharide estérifié, éthérifié ou un amido sucre.

**11.** Procédé selon l'une quelconque des revendications 1 à 10, effectué en présence d'un solvant, dans lequel le solvant ou les solvants sont choisis dans le groupe constitué par l'eau, les sulfoxydes, de préférence le DMSO, les cétones, de préférence la méthyléthylcétone, les liquides ioniques, la méthylisobutylcétone et/ou l'acétone, les esters, les éthers, de préférence les éthers d'éthylène glycol, de manière davantage préférée le diméthyl éther de diéthylène glycol (diglyme), ou le réactif oléfine et leurs mélanges.

**12.** Procédé selon l'une quelconque des revendications 1 à 11, dans lequel le procédé est effectué dans un procédé à écoulement continu, et dans lequel de préférence

a) le temps de séjour dans le procédé d'écoulement est compris entre 0,1 seconde et 10 heures, de préférence de 1 seconde à 1 heure, de manière davantage préférée de 5 secondes à 20 minutes ;
b) le procédé à écoulement continu est un procédé à écoulement continu à lit fixe ;
c) le lit fixe comprend un catalyseur acide hétérogène ;
d) le procédé à écoulement continu est un procédé de distillation réactive ou un procédé de distillation catalytique, et
e) en plus d'un catalyseur acide hétérogène, un catalyseur acide inorganique ou organique est ajouté à l'alimentation du procédé à écoulement continu à lit fixe ou de distillation catalytique.

**13.** Procédé selon la revendication 12, dans lequel la vitesse spatiale horaire liquide (« LHSV ») est de 1 à 1 000, de préférence de 5 à 500, de manière davantage préférée de 10 à 250 et de manière préférée entre toutes de 25 à 100.

**14.** Utilisation du mélange d'éthers produit par le procédé selon l'une quelconque des revendications 1 à 13, comme carburant ou additif de carburant.

**15.** Carburant ou composition de carburant comprenant le mélange d'éthers produit par le procédé de l'une quelconque des revendications 1 à 13, en tant que composant de carburant, facultativement mélangé avec un ou plusieurs éléments parmi l'essence et les mélanges essence-éthanol, le kérosène, le diesel, le biodiesel (un carburant diesel d'esters d'alkyle à chaîne courte (méthyle ou éthyle), non à base de pétrole et préparé par estérification d'une huile végétale), les liquides Fischer-Tropsch, les mélanges de diesel-biodiesel et le diesel vert (un hydrocarbure obtenu en hydrotraitant des huiles, des matières grasses, des graisses ou de l'huile de pyrolyse dérivées de la biomasse) et des mélanges de diesel et/ou de biodiesel avec du diesel vert et d'autres dérivés de furane et de tétrahydrofurane.

REFERENCES CITED IN THE DESCRIPTION

**Patent documents cited in the description**

- EP 0641854 A
- WO 2006063220 A
- EP 2007002145 W

- EP 0356703 A
- FR 2669634


**Non-patent literature cited in the description**

- **James A et al.** Phase modifiers promote efficient production of Hydroxymethylfurfural from fructose. *Science,* 30 June 2006, vol. 312 (5782), 1933-1937
- **Carlo Carlini.** Selective synthesis of 2-ethyl-1-hexanol from n-butanol through the Guerbet reaction by using bifunctional catalysts based on copper or palladium precursors and sodium butoxide. *Journal of Molecular Catalysis A: Chemical,* 2004, vol. 212, 65-70
- **Mr. Lewkowski.** Synthesis, chemistry and applications of 5-hydroxymethylfurfural and its derivatives. *Arkivoc.,* 2001, 17-54

- **Claude Moreau et al.** Dehydration of fructose and sucrose into 5-hydroxymethylfurfural in the presence of 1-H-3-methyl imidazolium chloride acting both as solvent and catalyst. *Journal of Molecular Catalysis A: Chemical,* 2006, vol. 253, 165-169
- *Adv. Synth. Catal.,* 2001, vol. 343, 220-225
- **Jerry March.** Advanced Organic Chemistry. John Wiley & Sons, 1985, 684-685
- **LEWKOWSKI, Jaroslaw.** Synthesis, chemistry and applications of 5-hydroxymethylfurfural and its derivatives. *Arkivoc.,* 2001, 17-54
- **MOREAU, Claude et al.** Dehydration of fructose and sucrose into 5-hydroxymethylfurfural in the presence of 1-H-3-methyl imidazolium chloride acting both as solvent and catalyst. *Journal of Molecular Catalysis A: Chemical,* 2006, vol. 253, 165-169